# EUROPEAN PATENT APPLICATION

(11) **EP 0 578 335 A2**
(43) Date of publication of application: **12.01.1994**
(21) Application number: 93202516.6
(22) Date of filing: 09.03.1989
(51) Int. Cl.: A61K 31/71

(54) **Combination of tobramycin and steroids for topical ophthalmic use**

(30) Priority: 09.03.1988 US 165950; 09.03.1988 US 165952
(62) Divisional of application: 89903311.2
(71) Applicant: ALCON LABORATORIES, INC., Fort Worth Texas 76134-2099 (US)
(72) Inventor: Cagle, Gerald D., Fort Worth Texas 76133 (US); McDonald, Thomas O., Fort Worth Texas 76107 (US); Rosenthal, Allan L., Fort Worth Texas 76109 (US)
(74) Representative: Kyle, Diana

(57) **Abstract**

Disclosed are pharmaceutical compositions comprising tobramycin and a steroid selected from fluorometholone and fluorometholone acetate for topical ophthalmic delivery and a method of treatment comprising administering said composition when indicated for infection and control of inflammatory response for optimal wound healing and normalization of the eye of a human or animal.

## Description

This invention relates to the ophthalmic use of antibiotics in combination with anti-inflammatory steroids when indicated for treatment of ophthalmic infections and attendant inflammation.

Formulations of antibiotics or steroids are available in the ophthalmic art. However, there are concerns and expressed reservations in the ophthalmic community about the safety and efficacy of such prior art combinations. There is, moreover a long felt need for an effective and safe topical ophthalmic pharmaceutical composition of a potent steroid and a broad spectrum antibiotic which, when administered to the eye when indicated for bacterial infection or as a prophylactic after ophthalmic trauma and injury, will not, as a possible expression of the steroid component, inhibit the activity of the antibiotic or interfere with normal wound healing, but at the same time will control inflammation.

According to this invention it has been discovered that the broad spectrum aminoglycoside antibiotic tobramycin in combination with a potent steroid meet these criteria.

The present invention provides topical ophthalmic compositions which comprise the combination of the aminoglycoside antibiotic tobramycin with a potent steroid selected from fluorometholone or its acetate.

The present invention provides topical ophthalmic compositions of matter which comprise mixtures of the broad spectrum aminoglycoside antibiotic tobramycin in combination with a potent steroid. The potent steroids according to the present invention are selected from fluorometholone or fluorometholone acetate. The amount of tobramycin and steroid will comprise a therapeutically effective amount of each substance in combination with a pharmaceutically acceptable carrier therefor. Preferably the ratio of tobramycin to steroid will range from 0.1 to 1.0 up to 10.0 to 1.0, respectively. Preferably, the mixture will contain about three times the weight of tobramycin to the weight of steroid.

Tobramycin is a water soluble, aminoglycoside antibiotic which is known chemically as 0-3 amino-3-deoxy-α-D-glucopyranosyl-(1→4)-0-[2,6-diamino-2,3,6-trideoxy-α-D-ribo-hexopyranosyl-(1→6)]-2-deoxy-L-streptamine. Tobramycin is reported as compound 9318 in the Merck Index, 10th Ed., 1983.

Fluoromethalone is a known anti-inflammatory glucocorticoid and its acetate and other esters are known from U.S. Patent No. 3,038,914. Fluoromethalone is described chemically as 9-fluoro-11,17-dihydroxy-6-methylpregna-1,4-diene-3,20-dione. It is listed as compound 4081 in the Merck Index, 10 Ed., 1983.

The compositions of the present invention comprise mixtures of the tobramycin and steroid in the amounts indicated. The composition will also contain other ingredients conventionally used in ophthalmic preparations such as antimicrobial preservatives, co-solvents, viscosity agents and the like.

The compositions of the present invention are administered topically to humans and animals. The dosage range is 0.001 to 5.0 mg/per eye, wherein the cited mass figures represent the sum of the two components, a steroid which is fluorometholone or fluoromethalone ester, and tobramycin. The compositions of the present invention can be administered as solutions, suspensions, or emulsions (dispersions) in a suitable ophthalmic vehicle.

In forming compositions for topical adminstration, the mixtures of antibiotic and steroid are preferably formulated as 0.01 to 2.0 percent by weight solutions in water at a pH of 4.5 to 8.0 (figures relate to combined presence of tobramycin and steroid). While the precise regimen is left to the discretion of the clinician, it is recommended that the resulting solution be topically applied by placing one drop in each eye of the human or animal two times a day.

### Antimicrobial Preservative:

Ophthalmic products are typically packaged in multidose form. Preservatives are thus required to prevent microbial contamination during use. Suitable preservatives include: benzalkonium chloride, thimerosal, chlorobutanol, methyl paraben, propyl paraben phenylethyl alcohol, edetate disodium sorbic acid, Onamer M, or other agents known to those skilled in the art. Typically such preservatives are employed at a level of from 0.001% to 1.0% by weight in the formulation.

### Co-Solvents:

The solubilty of the components of the present compositions may be enhanced by a surfactant or other appropriate co-solvent in the composition. Such co-solvents include polysorbate 20, 60, and 80, Pluronic F-68, F-84 and P-103, cyclodextrin, or other agents known to those skilled in the art. Typically such co-solvents are employed at a level of from 0.01% to 2% by weight in the formulation.

### Viscosity Agents:

Viscosity increased above that of simple aqueous solutions may be desirable to increase ocular absorption of the active compound, to decrease variability in dispensing the formulation, to decrease physical separation of components of a suspension or emulsion of the formulation and/or to otherwise improve the ophthalmic formulation. Such viscosity builder agents include as examples polyvinyl alcohol, polyvinyl pyrrolidone, methyl cellulose, hydroxy propyl methycellulose, hydroxyethyl cellulose, carboxymethyl cellulose, hydroxy propyl cellulose or other agents known to those skilled in the art. Such agents are typically employed at a level of from 0.01% to 2% by weight.

The combination of tobramycin and steroids such as fluoromethalone of the present invention provides a topical agent which takes advantage of the broad spectrum of activity of the tobramycin for ocular pathogens including some strains resistant to other antibiotics because it has a low potential for development of resistance and a lower incidence of sensitivity reactions than other antibiotics such as neomycin which are often used in steroid combinations.

In the combination pharmaceutical composition of this invention, it has been found that the tobramycin remains a highly effective antibiotic against susceptible strains of microorganisms such as staphylococci including S.aureus and S.epidermidis, including penicillin resistant strains, as well as streptococci including some of the group A species and some streptococcus pneumoniae. The combination pharmaceutical composition has been found effective in treating most external ocular infections caused by bacteria.

The compositions of the present invention are directed to the treatment of patients (i.e., "hosts") who exhibit the symptoms associated with ophthalmic infections and attendant inflammation, as well as to the treatment of inflammation in patients who may be predisposed to developing an ophthalmic infection, either as a result of an immunosuppressant effect attributable to the steroid component of the composition or as the result of a condition (e.g., injury) unrelated to the steroid therapy. The symptoms associated with these conditions are well known to ophthalmologists (i.e., medical doctors specializing in the treatment of eye disorders), as well as other physicians and clinicians having expertise in the treatment of ophthalmic infections and inflammation.

There has been a long felt need in the ophthalmic field for an improved, effective and safe combination of an antibiotic and an antiinflammatory steroid. In order to be both effective and safe, one component cannot interfere with or alter the action of the other component. For example, the steroid component must be capable of controlling inflammation without interfering with the action of the antibiotic. The present invention is based on the discovery that a particular combination, tobramycin and fluorometholone or fluorometholone acetate, satisfies these criteria.

The toxicology of the combination pharmaceutical composition of the invention has been shown to have a margin of safety equal to or greater than that found with other antibiotic-steroid combinations. Further, clinical efficacy studies found that the combination pharmaceutical compositions are safe and effective when used prior to and after ocular surgery.

As indicated above the composition of the present invention are particularly for use as ophthalmic compositions to be applied topically, preferably by administration of drops in the eye as clinically indicated The preferred composition comprises a composition which contains 0.3 weight percent of tobramycin and 0.1 weight percent of the steroid.

The mixtures are formed by conventional mixing of the required amounts of each of the active materials in combination with the other components desired to be included in the composition.

The following examples are representative pharmaceutical compositions of the invention for topical use when indicated against inflammation and infection. Parts are by weight unless otherwise indicated. The examples set forth preferred formulations.

### EXAMPLE I

| | | |
|---|---|---|
| Dexamethasone, Micronized USP | 1.0 mg + 5% excess | 0.10% + 5% excess |
| Tobramycin, USP | 3.0 mg + 5% excess | 0.30% + 5% excess |
| Benzalkonium Chloride Solution (10%), NF | 0.001 ml+10% excess | 0.10%+10% excess¹ |
| Edetate Disodium, USP | 0.1 mg | 0.01% |
| Sodium Chloride, USP | 3.0 mg | 0.3% |
| Sodium Sulfate, USP | 12.0 mg | 1.2% |
| Tyloxapol, USP | 0.5 mg | 0.05% |
| Hydroxyethylcellulose | 2.5 mg | 0.25% |
| Sulfuric Acid and/or Sodium Hydroxide, NF | QS for pH adjustment to 5.5 ± 0.5 | |
| Purified Water, USP | QS to 1 ml | QS to 100% |

| | | |
|---|---|---|
| ¹The benzalkonium chloride, NF concentration is equivalent to 0.01% (+ 10% excess). | | |

### EXAMPLE II

| | | |
|---|---|---|
| Dexamethasone, Micronized, USP | 0.1% + 2% excess | 1 mg + 2% excess |
| Tobramycin, Micronized, USP | 0.3% + 7% excess | 3 mg + 7% excess |
| Chlorobutanol, Anhydrous, NF | 0.5% + 25% excess | 5 mg + 15% excess |
| Mineral Oil, USP | 5% | 50 mg |
| White Petrolatum, USP | QS 100% | QS 1 g |

### EXAMPLE III

| | | |
|---|---|---|
| Fluorometholone Acetate, USP | 1.0 mg + 5% excess | 0.10% + 5% excess |
| Tobramycin, USP | 3.0 mg + 5% excess | 0.30% + 5% excess |
| Benzalkonium Chloride Solution (10%), NF | 0.001 ml+10% excess | 0.10%+10% excess¹ |
| Edetate Disodium, USP | 0.1 mg | 0.01% |
| Sodium Chloride, USP | 3.0 mg | 0.3% |
| Sodium Sulfate, USP | 12.0 mg | 1.2% |
| Tyloxapol, USP | 0.5 mg | 0.05% |
| Hydroxyethylcellulose | 2.5 mg | 0.25% |
| Sulfuric Acid and/or Sodium Hydroxide, NF | QS for pH adjustment to 5.5 ± 0.5 | |
| Purified Water, USP | QS to 1 ml | QS to 100% |

| | | |
|---|---|---|
| ¹The benzalkonium chloride, NF concentration is equivalent to 0.01% (+10% excess). | | |

### EXAMPLE IV

| | | |
|---|---|---|
| Fluorometholone Acetate, USP | 0.1% + 2% excess | 1 mg + 2% excess |
| Tobramycin, Micronized, USP | 0.3% + 7% excess | 3 mg + 7% excess |
| Chlorobutanol, Anhydrous, NF | 0.5% + 25% excess | 5 mg + 15% excess |
| Mineral Oil, USP | 5% | 50 mg |
| White Petrolatum, USP | QS 100% | QS 1 g |

### EXAMPLE V

### Clinical Efficacy

A controlled multi-center study was carried out with three investigators to ascertain the safety and efficacy of the ophthalmic composition of this invention relative to MAXITROL® ophthalmic suspension in the prevention of post-operative infection and in the control of post-operative inflammation after cataract surgery. The ophthalmic suspension of this invention was the formulation of Example 1 which contained .3 wt% of tobramycin and .1 wt% of dexamethasone. MAXITROL® is a commercially available multi-dose anti-infective steroid combination in sterile suspension for topical application. The active ingredient is dexamethasone in 0.1 wt% amounts. The other active ingredients are neomycin sulfate and Polymyxin sulfate. MAXITROL® is indicated for steroid responsive inflammatory ocular conditions for which a corticoid steroid is indicated and where bacterial infection or a risk of bacterial ocular infection exists. See Physician's Desk Reference for Ophthalmology, 12 Ed., 1984, p, 72. These studies were double masked and randomized. After the pressure enrollment examination, each patient was given a coded medication and instructed to dose one drop every four hours at home for three days prior to surgery. Post-surgical examinations were performed at days 1, 4, 7, 14 and 21. Dosing was as follows: 2 drops every 2 hours while awake for 2 days beginning with the first ocular dressing change the day after surgery; one drop four times per day for the next 7 days; 1 one drop daily for the next 10 days.

Of the 73 patients evaluated for efficacy, 36 patients received the composition of Example I and 37 were dosed with MAXITROL®. The two treatment groups did not differ in demographic characteristics, initial signs and symptoms, type of cataract, concomitant systemic medications and type of extraction. Surgically induced changes of the bulbur conjunctiva, palpervaral conjectiva and limbus were reported along with the following signs: aqueous reaction, erythema, epithelial disease, discharge, exudation, focal stromal infiltrates, iris damage and vitreous reaction. The results demonstrated no significant differences in the signs presented after surgery or in the rate of resolution of those signs.

Ocular infections did not occur in either treatment group. The effectiveness of the composition of this invention in controlling post-surgical inflammation after cataract surgery indicated effectiveness parallel to that of MAXITROL® for the control of post-surgical inflammation and demonstrated parallel effectiveness with regard to prophylaxis of post-operative infection and control of post-operative inflammation. Further, the composition of the invention was safe and effective when used prior to and after ocular surgery.

The invention has been described herein by reference to certain preferred embodiments. However, as obvious variations thereon will become apparent to those skilled in the art, the invention is not to be considered as limited thereto.

## Claims

1. An antibiotic/antiinflammatory ophthalmic pharmaceutical composition for topical delivery to the eye comprising a therapeutically effective amount of a mixture of tobramycin and a steroid selected from fluorometholone and fluorometholone acetate, and a pharmaceutically acceptable carrier therefor.

2. An antibiotic/antiinflammatory ophthalmic pharmaceutical composition according to claim 1 wherein the ratio of tobramycin to steroid is in the range of from 0.1 : 1.0 to 10.0 : 1.0.

3. A composition according to claim 2 wherein the steroid is fluorometholone acetate.

4. A composition according to claim 1 in the form of an agueous solution suspension, or emulsion containing 0.01 to 2.0 wt.% at a pH of 4.5 to 8.0.

5. A composition according to claim 4 which contains about 0.3 wt % tobramycin and about 0.1 wt % steroid.

6. A composition according to claim 1 which also contains one or bore preservatives, co-solvents, and viscosity builder agents.

7. Use of a therapeutically effective amount of a mixture of tobramycin and a steroid selected from fluoromecholone and fluorometholone acetate, for the manufacture of a combined antibiotic and antiinflammatory ophthalmic composition for topical delivery to the eye for treatment of inflammation and/or bacterial infection and/or as a prophylactic after ophthalmic trauma or injury.

8. Use according to claim 7, wherein the ratio of tobramycin to steroid is in the range of from 0.1 : 1.0 to 10.0 to 1.0.

9. Use according to claim 7, wherein the composition is in the form of an agueous solution, suspension, or emulsion containing 0.01 to 2.0 wt. % of the combined mixture of tobramycin and steroid at a pH of 4.5 to 8.0.

10. Use according to claim 9 which contains about 0.3 wt. % tobramycin and about 0.1 wt. % steroid.
